(19) Europäisches Patentamt
European Patent Office
Office européen des brevets

(11) EP 2 772 255 B1

(12) EUROPEAN PATENT SPECIFICATION

(45) Date of publication and mention of the grant of the patent:
**08.05.2019 Bulletin 2019/19**

(21) Application number: **12843151.7**

(22) Date of filing: **25.10.2012**

(51) Int Cl.:
*A61K 31/34* (2006.01) *A61K 8/49* (2006.01)
*A61P 17/00* (2006.01) *A61Q 19/00* (2006.01)
*A61Q 19/02* (2006.01) *A61Q 19/08* (2006.01)
*A61K 36/258* (2006.01) *A61K 8/97* (2017.01)

(86) International application number:
**PCT/KR2012/008816**

(87) International publication number:
**WO 2013/062333 (02.05.2013 Gazette 2013/18)**

(54) **COMPOSITION COMPRISING SYRINGARESINOL FOR IMPROVING THE SKIN**
ZUSAMMENSETZUNG MIT SYRINGARESINOL ZUR VERBESSERUNG DER HAUT
COMPOSITION COMPRENANT DU SYRINGARÉSINOL POUR AMÉLIORER LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2011 KR 20110110484**
**01.11.2011 KR 20110112764**

(43) Date of publication of application:
**03.09.2014 Bulletin 2014/36**

(73) Proprietor: **Amorepacific Corporation**
**Seoul 140-777 (KR)**

(72) Inventors:
- **SHIN, Hyun Jung**
**Yongin-si**
**Gyeonggi-do 446-729 (KR)**
- **CHO, Si Young**
**Yongin-si**
**Gyeonggi-do 446-729 (KR)**
- **SEO, Dae Bang**
**Yongin-si**
**Gyeonggi-do 446-729 (KR)**
- **PARK, Chan Woong**
**Yongin-si**
**Gyeonggi-do 446-729 (KR)**
- **KIM, Wan Gi**
**Yongin-si**
**Gyeonggi-do 446-729 (KR)**
- **LEE, Sang Jun**
**Yongin-si**
**Gyeonggi-do 446-729 (KR)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
**WO-A1-2004/000304** **WO-A1-2005/048970**
**DE-A1- 10 218 476** **JP-A- H0 913 031**
**JP-A- 2009 067 718** **JP-A- 2011 102 272**
**US-A1- 2007 166 255** **US-A1- 2009 130 154**

- **KI HYUN KIM ET AL: "Lignans from the Tuber-barks of Colocasia antiquorum var. esculenta and Their Antimelanogenic Activity", JOURNAL OF AGRICULTURAL AND FOOD CHEMISTRY, vol. 58, no. 8, 28 April 2010 (2010-04-28) , pages 4779-4785, XP055191222, ISSN: 0021-8561, DOI: 10.1021/jf100323q**
- **WANG H.-M. ET AL.: 'Bioconstituents from stems of Synsepalum dulcificum Daniell (Sapotaceae) inhibit human melanoma proliferation, reduce mushroom tyrosinase activity and have antioxidant properties' JOURNAL OF THE TAIWAN INSTITUTE OF CHEMICAL ENGINEERS vol. 42, March 2011, pages 204 - 211, XP003031611**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **YAMAZAKI T. ET AL.: '(+)-Syringaresinol-di-O-beta-D-glucoside, a phenolic compound from Acanthopanax senticosus Harms, suppresses proinflammatory mediators in SW982 human synovial sarcoma cells by inhibiting activating protein-1 and/or nuclear factor-kB activities' TOXICOLOGY IN VITRO vol. 21, 2007, pages 1530 - 1537, XP022340102**

Remarks:

The file contains technical information submitted after the application was filed and not included in this specification

## Description

[Technical Field]

The present disclosure relates to the cosmetic use of syringaresinol for improving skin.

[Background Art]

The color of human skin is determined by various factors including the activity of melanin-producing melanocytes, the distribution of blood vessels, the thickness of skin, the presence of pigments such as carotenoids, bilirubin, etc., or the like. Among them, the production of melanin by melanocytes mediated by many enzymes including tyrosinase is one of the most important factors that determine the skin color.

Melanin present in skin plays an important role of protecting against UV, etc. However, in excess, it is known to induce skin pigmentations such as melasma, freckle, lentigo, etc., accelerate skin aging and cause skin cancer.

In order to treat or alleviate skin pigmentation caused by hyperproduction of melanin, various substances exhibiting skin whitening effects, such as ascorbic acid, kojic acid, arbutin, hydroquinone, glutathione or derivatives thereof, have been used. But, they are limited in applications because of imperfect skin whitening effect, unguaranteed safety, difficulty in formulation as cosmetics and stability problem.

Skin is the part of the body which is the most vulnerable to UV because it is directly exposed to sunlight. Upon exposure to UV, the skin exhibits biological responses such as burn, pigmentation, DNA damage, change in connective tissues, and cancer, depending on the degree. Also, prolonged exposure to UV causes early skin aging, thereby leading to wrinkle formation, pigmentation, change in connective tissues, change in the thickness of the epidermis, and so forth.

The connective tissue of the skin mainly consists of collagen and elastin. Since collagen and elastin give elasticity to the skin, the skin is easily damaged and ages if they are weakened. Matrix metalloproteinases (MMPs) are enzymes that are involved in the breakdown of collagen. As the skin ages, the expression of MMPs increases and the increased MMPs break down the skin collagen. As this mechanism is repeated, the skin develops wrinkles and ages earlier.

Transforming growth factor β (TGF-β) is a substance involved in the growth and differentiation of various cells. In mammals, TGF-β exists in three different isoforms. Among them, TGF-β1 exhibits the strongest activity for the formation of extracellular matrix (ECM) in the dermis and is involved in the restoration and differentiation of tissues. TGF-β1 produces various ECM components such as type I and type II collagen, fibronectin, proteoglycan, etc. by stimulating fibroblasts. Accordingly, a substance that facilitates the production of TGF-β1 may be able to prevent skin aging and improve skin wrinkles by promoting the growth of fibroblasts and accelerating skin regeneration.

To keep the skin moisturized, it is necessary to increase natural moisturizing factors in the skin. Since amino acids as the major component of the natural moisturizing factors are supplied from the breakdown of the protein filaggrin produced by keratinocytes, promoted filaggrin production may result in skin moisturizing effect. Also, since involucrin is a factor that plays a critical role in the formation of a cell envelope that prevents skin loss and the structuralization of the horny layer, promoted expression thereof may also lead to superior skin moisturizing effect.

WO 2005/048970 discloses that a group lignans, comprising syringaresinol besides seven others compounds, can be used in cosmetics.

JP 2011-102272 A discloses use of Yangambin for the maturation of the blood vessel, normalization or stabilization medicine, the lymphatic vessel stabilization medicine, and the wrinkle.

WO 2004/000304 A1 discloses that a group lignans, comprising syringaresinol besides twelve others compounds, for treating dermatological aging and skin cancer.

KI HYUN KIM et al. ("Lignans from the Tuber-barks of Colocasia antiquorum var. esculenta and their antimelanogenic activity", Journal of agricultural and food chemistry, vol. 58, no. 8, 28 April 2010, pages 4779-4785) relates to skin whitening agents by inhibiting a melanin formation.

DE 102 18476 A1 discloses sesamine and pinoresinol for treating skin aging.

US 2009/130154 A1 discloses a large number of metal zeolite compounds, among which metal zeolite syringaresinol is cited, and their use in very numerous and different skin conditions to be treated, including skin rash; darkened skin; skin aging; body odor; cellular inflammation; premature hair aging and malfunction of tyrosinase group of enzymes.

US 2007/166255 A1 discloses a topical treatment of topical discomforts and ailments including sunburn, radiation burn, thermal burn and blisters, diaper rash, shaving burn, wound, venous cannulation, acne, darkened skin pigmentation, skin wrinkles, dandruff, and hair loss, said treatment achieved via topical inhibition of Fatty Acid Desaturases.

WANG H.-M. et al. ("Bioconstituents from stems of Synsepalum dulcificum Daniell (Sapotaceae) inhibit human melanoma proliferation, reduce mushroom tyroninase activity and have antioxidant properties", Journal of the Taiwan institute of chemical engineers, vol. 42, March 2011, pages 204-211) discloses inhibition effect on human melanoma cells.

Yamazaki et al. ("(+)-Syringaresinol-di-O-beta-D-glucoside, a phenolic compound from Acanthopanax senticosus Harms, suppresses proinflammatory mediators in SW982 human synovial sarcoma cells by inhibiting activating protein-1 and/or nuclear factor-kB activities", Toxicology in vitro, vol. 21, 2007, pages 1530-1537) describes the effect of syringaresinol-di-O-beta-D-glucoside as MMP inhibitor and its use as antiinflammatory agent for treating arthritis.
JP 2009 067718 A teaches the use of a preparation containing syringaresinol and cathechin as having active oxygen dismutase-like (SOD-like) activity, for preventing aging spot and wrinkles.
JP H09 13031 discloses the use of Syringaresinol-di-O-beta-D-glucoside as antioxidant in medicine and cosmetics.

**[Disclosure]**

The present disclosure is directed to providing a cosmetic use of syringaresinol for facilitating keratin removal and keratin turnover, promoting skin regeneration or promoting skin moisturization.
The present disclosure provides a cosmetic use of syringaresinol, for facilitating keratin removal and keratin turnover, promoting skin regeneration or promoting skin moisturization, with exclusion of any therapeutic use.
In the cosmetic use of the invention, the syringaresinol is included in an extract of one or more selected from flax seed, phellodendri cortex, acanthopanacis cortex, sesame seed and ginseng berry.
In the cosmetic use of the invention, the compound or the salt thereof is administered in a form of a cosmetic composition.
In the cosmetic use of the invention, the composition comprises 0.001-20 wt% of the compound or the salt thereof based on the total weight of the composition.

[Brief Description of Drawings]

Fig. 1 schematically describes a method of isolating and purifying syringaresinol from a ginseng berry extract.
Fig. 2 compares the free radical scavenging ability of syringaresinol with vitamin C.
Fig. 3 shows the SIRT1 expression promoting effect of syringaresinol in keratinocytes.
Fig. 4 shows the SIRT1 expression promoting effect of syringaresinol in fibroblasts.
Fig. 5 shows the filaggrin and involucrin expression promoting effect of syringaresinol.
Fig. 6 shows the MMP-9 expression and collagen breakdown inhibiting effect of syringaresinol.
Fig. 7 shows the skin wrinkle inhibiting effect of syringaresinol upon application or oral administration.
Fig. 8 shows the transepidermal water loss (TEWL) inhibiting effect of syringaresinol upon application or oral administration.
Fig. 8 shows the transepidermal water loss (TEWL) inhibiting effect of syringaresinol upon application or oral administration.

In the present disclosure, the term "extract" is used as a broad concept and refers to any substance extracted from a natural product, regardless of extraction method, extraction solvent, extracted ingredients or the type of extract.
As used herein, the term "skin" refers to a tissue that covers the body surface of an animal and is used in the broadest concept, including not only the tissue that covers the surface of face or body but also the scalp and hair.
As used herein, the term "improvement" refers to any positive effect on normal or abnormal skin, including moisturization, wrinkle improvement, etc. Specifically, the composition of the present disclosure may provide skin improving effect by enhancing skin whitening or preventing aging, although not being limited thereto.
Hereinafter, the present disclosure is described in detail.
Hereinafter, the present disclosure is described in detail.
In an aspect, the present disclosure provides, a cosmetic use of syringaresinol, for facilitating keratin removal and keratin turnover, promoting skin regeneration or promoting skin moisturization, with exclusion of any therapeutic use.
Syringaresinol may be synthesized chemically or extracted from one or more of flax seed, phellodendri cortex, acanthopanacis cortex, sesame seed and ginseng berry. The flax seed, parts of the plant, for example, leaves, stem, root, fruit or seed and the ginseng berry includes the rind or pulp of ginseng berry.

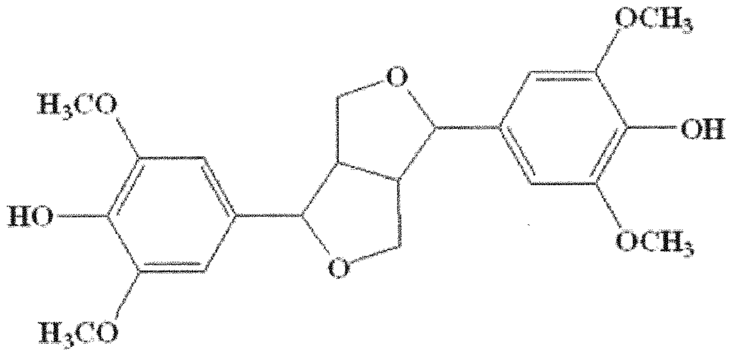

In the present disclosure, the "syringaresinol" may be obtained by extracting one or more of flax seed, phellodendri cortex, acanthopanacis cortex, sesame seed and ginseng berry with water, an organic solvent or a mixture of water and an organic solvent. The organic solvent includes one or more selected from a group consisting of alcohol, acetone, ether, ethyl acetate, diethyl ether, methyl ethyl ketone and chloroform, although not being limited thereto. The alcohol includes a $C_1$-$C_5$ lower alcohol and the $C_1$-$C_5$ lower alcohol includes one or more selected from a group consisting of methanol, ethanol, isopropyl alcohol, n-propyl alcohol, n-butanol and isobutanol, although not being limited thereto.

In an exemplary embodiment of the present disclosure, syringaresinol may be isolated and purified from ginseng berry by a procedure including: preparing an alcohol extract of ginseng berry pulp; eluting the prepared alcohol extract with a solvent including one or more of water and alcohol and obtaining fractions thereof; and performing chromatography, specifically thin-layer chromatography (TLC), on the obtained fractions using an organic solvent as an eluent. The organic solvent may include one or more selected from a group consisting of alcohol, acetone, ether, may include one or more selected from a group consisting of alcohol, acetone, ether, ethyl acetate, diethyl ether, methyl ethyl ketone and chloroform, and the alcohol may include a $C_1$-$C_5$ alcohol. In an exemplary embodiment of the present disclosure, the composition may contain the syringaresinol purified as described above as an active ingredient.

In an exemplary embodiment of the present disclosure, the composition may contain the active ingredient in an amount of 0.001-20 wt%, specifically 0.01-10 wt%, more specifically 0.1-5 wt%, based on the total weight of the composition. This range is appropriate not only to derive the effect desired by the present disclosure and satisfy both the stability and safety of the composition but also in terms of cost effectiveness. Specifically, if the content of the active ingredient is less than 0.01 wt%, sufficient skin whitening effect may not be achieved. And, if it exceeds 20 wt%, the safety and stability of the composition may be unsatisfactory.

[Mode for Invention]

Hereinafter, the present disclosure will be described in detail through an example and test examples. However, the following example and test examples are for illustrative purposes only and it will be apparent to those of ordinary skill in the art that the scope of the present disclosure is not limited by the example and test examples.

**[Example] Isolation and analysis of syringaresinol**

Live ginseng berry was harvested. After removing the seed and rind of ginseng berry, only the pulp was dried under sunlight or using hot air to obtain dried ginseng berry pulp.

**2. Isolation of syringaresinol from ginseng berry pulp extract and analysis thereof**

3 L of water or alcohol was added to 1 kg of the dried ginseng berry pulp. After extracting at room temperature or under reflux, followed by filtering and concentration at 40-45 °C under reduced pressure, 300 g of a ginseng berry pulp extract was obtained. The extract was treated with ether to remove oil-soluble components and then crude saponin was extracted with butanol and concentrated. Then, syringaresinol was isolated and purified therefrom as follows. First, 194 g of the sample was purified by reversed-phase (ODS) column chromatography. As the eluent, 100% water was used in the beginning. Subsequently, methanol was increased gradually by 10% and, finally, 100% methanol was used as the eluent. As a result, fractions GB-1 through GB-10 were obtained. Among the fractions, the fraction GB-3 which exhibits sirtuin 1 (SIRT1) expression activity was concentrated and subjected to Sephadex LH-20 column chromatography using 50% aqueous methanol. Among the resulting fractions, the fraction GB-3-6(3F) exhibiting SIRT1 expression activity was concentrated and subjected to preparative silica gel TLC using chloroform:methanol (10:1) as an eluent. As a result, an active fraction with an $R_f$ value of 0.67 was purified. This procedure is schematically described in Fig. 1.

Through NMR spectroscopic analysis and database search, the isolated and purified active compound was identified as syringaresinol. Mass analysis was conducted to confirm this. As a result of ESI-mass analysis in the positive mode, $[M+Na]^+$ (m/z = 440.9) and $[2M+Na]^+$ (m/z = 858.9) peaks were observed and the molecular weight was calculated as 418. And, the result of NMR spectroscopic analysis was as in Chemical Formula 3. Accordingly, the isolated and purified active compound was confirmed to be syringaresinol.

【Chemical Formula 3】

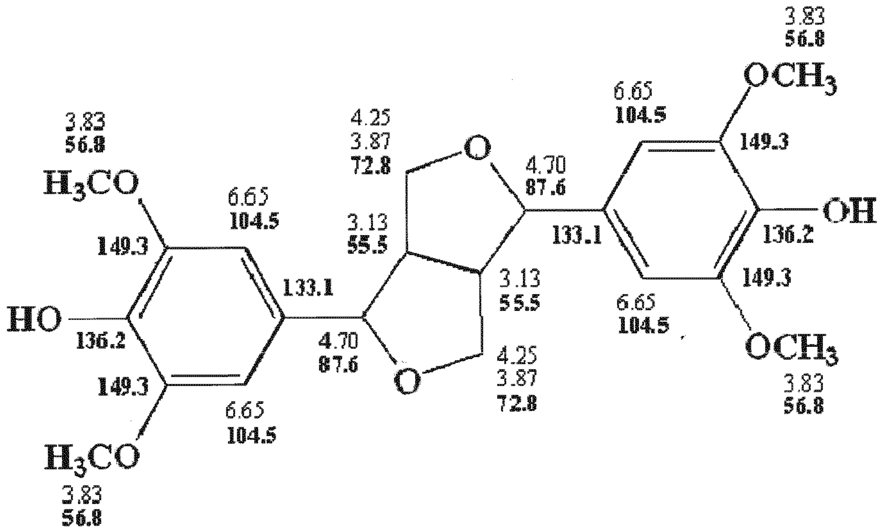

As such, syringaresinol was isolated from the ginseng berry pulp.

[Test Example 1] Test of cytotoxicity

Cytotoxicity was tested for syringaresinol in mouse melanocytes as follows.

Melanocytes (Mel-Ab cells) derived from C57BL/6 (treated with Dooley et al.'s method) were cultured under the condition of 37 °C and 5% $CO_2$ in Dulbecco's modified Eagle's medium (DMEM) containing 10% fetal bovine serum, 100 nM 12-0-tetradecanoylphorbol-13-acetate and 1 nM cholera toxin. The cultured Mel-Ab cells were detached with 0.25% trypsin-EDTA, cultured in a 24-well plate to a concentration of $10^5$ cells/well and then treated with the syringaresinol prepared in Example. After culturing for 24 hours, the cells were washed with phosphate buffered saline (PBS, pH 7.2) and then stained using 0.1% crystal violet (Sigma) and 10% ethanol (EtOH) for 5 minutes at room temperature. After completely removing free crystal violet, the cells were washed 3 times with PBS (pH 7.2). The stained cells were treated with 95% ethanol (EtOH) for 1 hour at room temperature to extract crystal violet. The absorbance of the extract was measured at 540 nm using the Lambda 25 spectrophotometer (PerkinElmer) and cell viability (%) was calculated according to Equation 1. The result is shown in Table 1.

【Equation 1】

$$\text{Cell viability (\%)} = (A / B) \times 100$$

A: absorbance in the presence of syringaresinol
B: absorbance in the absence of syringaresinol

[Table 1]

| Test substance | Cell viability (%) |
|---|---|
| Syringaresinol | 98.6 |

As seen from Table 1, syringaresinol can increase cell viability by significantly reducing the toxicity of skin cells.

**[Test Example 10] Evaluation of SIRT1 expression promoting effect in human keratinocytes and fibroblasts**

The following experiment was carried out to evaluate the SIRT1 gene expression promoting effect of syringaresinol in human keratinocytes and fibroblasts.

**1. Cell lines and cell culturing**

Human keratinocyte HaCaT cells or human fibroblast HDF cells were cultured under the condition of 37 °C and 5% $CO_2$ in DMEM ((Gibco 1210-0038)) containing 10% fetal bovine serum.

**2. Evaluation of SIRT1 expression promoting effect**

The cultured cells were treated with syringaresinol dissolved in DMSO at a concentration of 20, 50 or 100 μM for 24 hours. Separately from this, the cells were treated with DMSO of 1/1000 of the volume of the culture medium as a negative control. After treating with the test substance, the cells were washed twice with cold PBS and RNA was extracted using TRIzol reagent (Invitrogen). Then, cDNA was synthesized using 1 μg/μL of the extracted RNA and a reverse transcription system (Promega). The expression pattern of the SIRT1 and GAPDH genes was monitored using the synthesized cDNA and predesigned primers and probes (Applied Biosystems; SIRT1, Hs01009006_m1; GAPDH, Hs99999905_m1). PCR reaction and analysis were carried out using the Rotor-Gene 3000 system (Corbett Research, Sydney, Australia). The result is shown in Fig. 3 (keratinocytes) and Fig. 4 (fibroblasts).

As seen from Fig. 3 and Fig. 4, syringaresinol can increase the expression of SIRT1 in human keratinocytes and fibroblast by 4 times or more in a concentration-dependent manner.

**Evaluation of filaggrin and involucrin expression promoting effect in human keratinocytes**

The following experiment was carried out to evaluate the filaggrin and involucrin expression promoting effect of syringaresinol in human keratinocytes.

**1. Cell line and cell culturing**

Human keratinocyte HaCaT cells were cultured in substantially the same manner as in Test Example 10.

**2. Evaluation of filaggrin and involucrin expression promoting effect**

cDNA was synthesized in substantially the same manner as in Test Example 10 and the expression pattern of the filaggrin, involucrin and GAPDH genes was monitored substantially the same manner as in Test Example 10 using the synthesized cDNA and predesigned primers and probes (Applied Biosystems; SIRT1, Hs01009006_m1; GAPDH, Hs99999905_m1). The result is shown in Fig. 5.

As seen from Fig. 5, syringaresinol increases the expression of filaggrin and involucrin in human keratinocytes in a concentration-dependent manner. Accordingly, it can be seen that syringaresinol enhances skin barrier function and exhibits skin moisturizing effect.

**Evaluation of MMP-9 expression and collagen breakdown inhibiting effect in human keratinocytes**

The following experiment was carried out to evaluate the MMP-9 expression and collagen breakdown inhibiting effect of syringaresinol in human keratinocytes.

**1. Cell line and cell culturing**

Human keratinocyte HaCaT cells were cultured in substantially the same manner as in Test Example 10.

**2. Evaluation of UV-induced MMP-9 expression and collagen breakdown inhibiting effect**

The cultured cells were treated with trypsin to obtain a single-cell suspension. Then, the cells were distributed in a 6-well plate, with $2x10^5$ cells per well, and cultured for 24 hours. Subsequently, after replacing the medium with DMEM not containing fetal bovine serum and culturing again for 24 hours, the cells were treated with 50 μM syringaresinol

dissolved in DMSO. After treating for 24 hours, the cells were washed with PBS and UVB of 30 mJ/$cm^2$ was irradiated in PBS. After discarding the PBS, the cells were added to a medium containing syringaresinol of the same concentration. As a control group, the cells were cultured in the same manner without treating with syringaresinol. 24 hours after the UV radiation, the cells treated and untreated with syringaresinol were washed with PBS, recovered by treating with trypsin, treated with 500 mL of protein extraction buffer (8 M urea, 2% CHAPS, 50 mM DTT, 2 M thiourea, 2 mM PMSF, 100 mg/mL leupeptin) and kept at room temperature for 10 minutes. Then, after centrifuging at 4 °C for 10 minutes with 15,000 g, the supernatant was recovered and protein was quantitated using Bio-Rad Protein Dye Reagent™. 20 mg of protein was fractionated by size using 8% SDS-PAGE and blotted on PDF membrane (Bio-Rad) for 12 hours with 50 V. The resulting blot was blocked for 1 hour with 5% non-fat milk and reacted using monoclonal anti-MMP-9 antibody (Santa Cruz, CA, USA) and monoclonal anti-collagen IV antibody (Abcam, Cambridge, MA, USA) as primary antibodies, horse radish peroxidase-conjugated anti-mouse IgG (Amersham) as secondary antibody and the enhanced chemiluminescence (ECL) kit (Amersham). The reacted blot was exposed to X-ray Fuji film and developed to investigate the protein expression level. Bands on the film were scanned with PowerLook 2100 XL (UMAX) and analyzed with the image-analyzing program ImageMaster 2D Elite (Amersham Bioscience). The result is shown in Fig. 6.

As seen from Fig. 6, UV radiation leads to increased MMP-9 expression and decreased collagen as a result of facilitated collagen breakdown in keratinocytes. However, treatment with syringaresinol inhibits collagen breakdown by inhibiting the UV-induced increase of MMP-9 expression, thereby preventing UV-induced decrease in collagen production. That is to say, syringaresinol can prevent and improve skin wrinkles by inhibiting MMP-9 expression and collagen breakdown and thus can prevent skin aging.

**Evaluation of TGF-β1 expression promoting effect in fibroblasts**

The following experiment was carried out to evaluate the TGF-β1 expression promoting effect of syringaresinol in fibroblasts.

**1. Cell line and cell culturing**

Human fibroblast HDF cells were cultured in substantially the same manner as in Test Example 10.

**2. Evaluation of TGF-β1 expression promoting effect**

The cultured cells were treated with syringaresinol dissolved in DMSO to a concentration of 20, 50 or 100 μM for 24 hours. After 24 hours, the cell culture was recovered.

The quantity of TGF-β1 produced in the recovered cell culture was measured using the Quantikine™ high-sensitivity ELISA kit (R&D Systems, USA) according to the manufacturer's instructions. All the samples required for the measurement of the quantity of TGF-β1 were provided by the kit. First, the recovered cell culture was transferred to a 96-well microplate uniformly coated with TGF-β1 antibody and it was allowed for antigen-antibody reaction to proceed at 37 °C for 1 hour. After removing the cell culture, the wells were washed 3 times with PBS. Then, horseradish peroxidase-conjugated secondary antibody was added to each well and it was allowed for antigen-antibody reaction to proceed for 1 hour. Subsequently, after adding tetramethylbenzidine as a chromogenic substrate to each well, followed by reaction at room temperature for 15 minutes, 1 N sulfuric acid was added to stop the reaction. Then, absorbance was measured at 450 nm using a spectrophotometer. After drawing a standard curve using a standard solution, the TGF-β1 production amount in the cell culture with the test substance added was calculated from the measured absorbance. The result is shown in Table 10.

[Table 10]

| Syringaresinol (μM) | TGF-β1 |
|---|---|
| 0 | 23.7±1.2 |
| 20 | 43.3±1.0 |
| 50 | 87.2±4.2 |
| 100 | 92.1±5.6 |

As seen from Table 10, syringaresinol increases the production of TGF-β1 in fibroblasts in a concentration-dependent manner. Accordingly, it can be seen that syringaresinol can promote skin regeneration by increasing TGF-β1 production and thus can prevent skin aging and improve skin wrinkles.

**Evaluation of wrinkle formation and transepidermal water loss inhibiting effect in hairless mouse skin**

The following experiment was carried out to evaluate the wrinkle formation and transepidermal water loss inhibiting effect of syringaresinol.

**1. Treatment of hairless mouse**

30-week-old female hairless mice (SKH: HR-1) weighing 25-39 g were purchased from Charles River Laboratories (Wilmington, MA, USA). The mice were accustomed to the laboratory environment for a week and then grouped into normal groups and test substance-treated groups, with 5 mice per each group. The mice were allowed free access to water and feed under the condition of 12-hour light/dark cycles, 23.2 °C and 55.10% humidity.

**2. Evaluation of skin wrinkle formation inhibiting effect of syringaresinol when applied on skin or orally administered**

For the test substance-treated groups, a patch of 1% syringaresinol dissolved in a solvent (1,3-butylene glycol:ethanol = 7:3) was applied 2 times for 2 days or 200 mg/kg syringaresinol was orally administered. The control groups were treated in the same way using the solvent of the same amount instead of syringaresinol. The syringaresinol-applied group, syringaresinol oral administration group and control groups were exposed to UVB of 180 $mJ/cm^2$. Before and after the UV radiation, skin wrinkles on the exposed part were taken with replica and measured using the Visiometer system (C+K). The change in skin wrinkles was calculated according to Equation 4 and the result is shown in Fig. 7.

【Equation 4】

$$\text{Change (\%)} = [(T_{di} - T_{do}) / (T_{do})] \times 100$$

$T_{di}$: value measured after UV radiation
$T_{do}$: value measured before UV radiation

As seen from Fig. 7, the application and oral administration control groups show increase of skin wrinkles by 230±35% and 224±45%, respectively, whereas the syringaresinol application and oral administration groups show only slight increase of 121±35% and 123±25%, respectively. Accordingly, it can be seen that syringaresinol has very superior skin wrinkle inhibiting effect.

**3. Measurement of transepidermal water loss (TEWL)**

3 weeks after the onset of experiment, transepidermal water loss (TEWL) was measured on the back part of the hairless mouse of each group using a wireless vapometer (Delfin Technologies, Ltd., SWL 4102). The result is shown in Fig. 8. The measurement environment was 60±5% relative humidity and 25.5±0.5 °C.

Transepidermal water loss is the quantity of water that passes from inside the body through the skin and a larger value means poor skin moisturization ability and damaged skin barrier function. Accordingly, the transepidermal water loss may be used as an index in evaluating skin barrier function, recovery of damaged skin barrier function, protection of skin from stimulations such as UV, and so forth.

As seen from Fig. 8, UV radiation results in deteriorated skin moisturization ability because of damaged skin barrier function. Oral administration or application of syringaresinol leads to decrease in UV-induced transepidermal water loss. Accordingly, it can be seen that syringaresinol can provide superior skin barrier function recovering, skin protecting and skin moisturizing effect by preventing UV-induced transepidermal water loss.

Hereinafter, the present disclosure will be described in detail through formulation examples. However, the formulation examples are for illustrative purposes only and it will be apparent to those of ordinary skill in the art that the scope of the present disclosure is not limited by the formulation examples.

[Formulation Example 1] Lotion

A lotion is prepared according to a commonly employed method with the following composition.

[Table 11]

| Ingredients | Content (wt%) |
|---|---|
| Syringaresinol | 3.0 |
| L-Ascorbic acid-2-phosphate magnesium salt | 1.0 |
| Water-soluble collagen (1% aqueous solution) | 1.0 |
| Sodium citrate | 0.1 |
| Citric acid | 0.05 |
| 1,3-Butylene glycol | 3.0 |
| Purified water | balance |

[Formulation Example 2] Cream

A cream is prepared according to a commonly employed method with the following composition.

[Table 12]

| Ingredients | Content (wt%) |
|---|---|
| Syringaresinol | 1.0 |
| Polyethylene glycol monostearate | 2.0 |
| Glycerin monostearate | 5.0 |
| Cetyl alcohol | 4.0 |
| Squalene | 3.0 |
| Glyceryl tri(2-ehtylhexanoate) | 6.0 |
| Sphingolipid | 1.0 |
| 1,3-Butylene glycol | 7.0 |
| Purified water | balance |

[Formulation Example 3] Pack

A pack is prepared according to a commonly employed method with the following composition.

[Table 13]

| Ingredients | Content (wt%) |
|---|---|
| Syringaresinol | 5.0 |
| Polyvinyl alcohol | 13.0 |
| L-Ascorbic acid-2-phosphate magnesium salt | 1.0 |
| Lauroyl hydroxyproline | 1.0 |
| Water-soluble collagen (1% aqueous solution) | 2.0 |
| 1,3-Butylene glycol | 3.0 |
| Ethanol | 5.0 |
| Purified water | balance |

[Formulation Example 4] Soft capsule

After mixing 100 mg of syringaresinol, 50 mg of soybean extract, 180 mg of soybean oil, 50 mg of red ginseng

extract, 2 mg of palm oil, 8 mg of hydrogenated palm oil, 4 mg of yellow beeswax and 6 mg lecithin, a soft capsule is prepared according to a commonly employed method.

[Formulation Example 5] Tablet

Granules formed by mixing 100 mg of syringaresinol g, 50 mg of soybean extract, 100 mg of glucose, 50 mg of red ginseng extract, 96 mg of starch and 4 mg of magnesium stearate and adding 40 mg of 30% ethanol are dried and prepared into a tablet.

[Formulation Example 6] Health food

| | |
|---|---|
| Syringaresinol | 1000 mg |
| Vitamin mixture | |
| Vitamin A acetate | 70 μg |
| Vitamin E | 1.0 mg |
| Vitamin $B_1$ | 0.13 mg |
| Vitamin $B_2$ | 0.15 mg |
| Vitamin $B_6$ | 0.5 mg |
| Vitamin $B_{12}$ | 0.2 μg |
| Vitamin C | 10 mg |
| Biotin | 10 μg |
| Nicotinamide | 1.7 mg |
| Folic acid | 50 μg |
| Calcium pantothenate | 0.5 mg |
| Mineral mixture | |
| Ferrous sulfate | 1.75 mg |
| Zinc oxide | 0.82 mg |
| Magnesium carbonate | 25.3 mg |
| Potassium dihydrogen phosphate | 15 mg |
| Calcium monohydrogen phosphate | 55 mg |
| Potassium citrate | 90 mg |
| Calcium carbonate | 100 mg |
| Magnesium chloride | 24.8 mg |

Although the above-described mixing ratios of the vitamin and mineral mixtures are provided as specific examples suitable for health food, the mixing ratios may be changed as desired.

[Formulation Example 7] Health drink

| | |
|---|---|
| Syringaresinol | 1000 mg |
| Citric acid | 1000 mg |
| Oligosaccharide | 100 g |
| Taurine | 1 g |
| Purified water | balance |

According to a commonly employed method, the above-described ingredients are mixed and stirred for about 1 hour while heating at about 85 °C. The resulting solution is filtered and sterilized.

## Claims

1. Cosmetic use of syringaresinol or the salt thereof for facilitating keratin removal and keratin turnover, promoting skin regeneration or promoting skin moisturization, with exclusion of any therapeutic use.

2. The cosmetic use according to claim 1, wherein the syringaresinol or the salt thereof is included in an extract of one or more selected from flax seed, phellodendri cortex, acanthopanacis cortex, sesame seed and ginseng berry.

3. The cosmetic use according to any one of claims 1 or 2, wherein the syringaresinol or the salt thereof is administered in a form of a cosmetic composition.

4. The cosmetic use according to claim 3, wherein the composition comprises 0.001-20 wt% of the syringaresinol or the salt thereof based on the total weight of the composition.

## Patentansprüche

1. Kosmetische Verwendung von Syringaresinol oder dem Salz davon zur Vereinfachung der Entfernung von Keratin und des Stoffumsatzes von Keratin, Förderung der Hautregenerierung oder Förderung der Hautbefeuchtung mit Ausnahme jeder therapeutischen Verwendung.

2. Kosmetische Verwendung nach Anspruch 1, wobei das Syringaresinol oder das Salz davon in einen Extrakt von einem oder mehreren eingeschlossen ist, ausgewählt aus Leinsamen, Cortex phellodendri, Cortex acanthopanacis, Sesamkörnern und Ginseng-Beere.

3. Kosmetische Verwendung nach einem der Ansprüche 1 oder 2, wobei das Syringaresinol oder das Salz davon in einer Form einer kosmetischen Zusammensetzung verabreicht wird.

4. Kosmetische Verwendung nach Anspruch 3, wobei die Zusammensetzung 0,001 bis 20 Gew% des Syringaresinols oder des Salzes davon umfasst, basierend auf dem Gesamtgewicht der Zusammensetzung.

## Revendications

1. Utilisation cosmétique de syringarésinol ou du sel de celui-ci pour faciliter l’élimination de la kératine et le renouvellement de la kératine, favoriser la régénération de la peau ou favoriser l’hydratation de la peau, à l’exclusion de toute utilisation thérapeutique.

2. Utilisation cosmétique selon la revendication 1, dans laquelle le syringarésinol ou le sel de celui-ci est inclus dans un extrait d’un ou de plusieurs sélectionnés parmi la graine de lin, le cortex phellodendri, le cortex acanthopanacis, la graine de sésame et la baie de ginseng.

3. Utilisation cosmétique selon l’une quelconque des revendications 1 ou 2, dans laquelle le syringarésinol ou le sel de celui-ci est administré sous la forme d’une composition cosmétique.

4. Utilisation cosmétique selon la revendication 3, dans laquelle la composition comprend 0,001 à 20 % en poids du syringarésinol ou du sel de celui-ci sur la base du poids total de la composition.

Fig.1

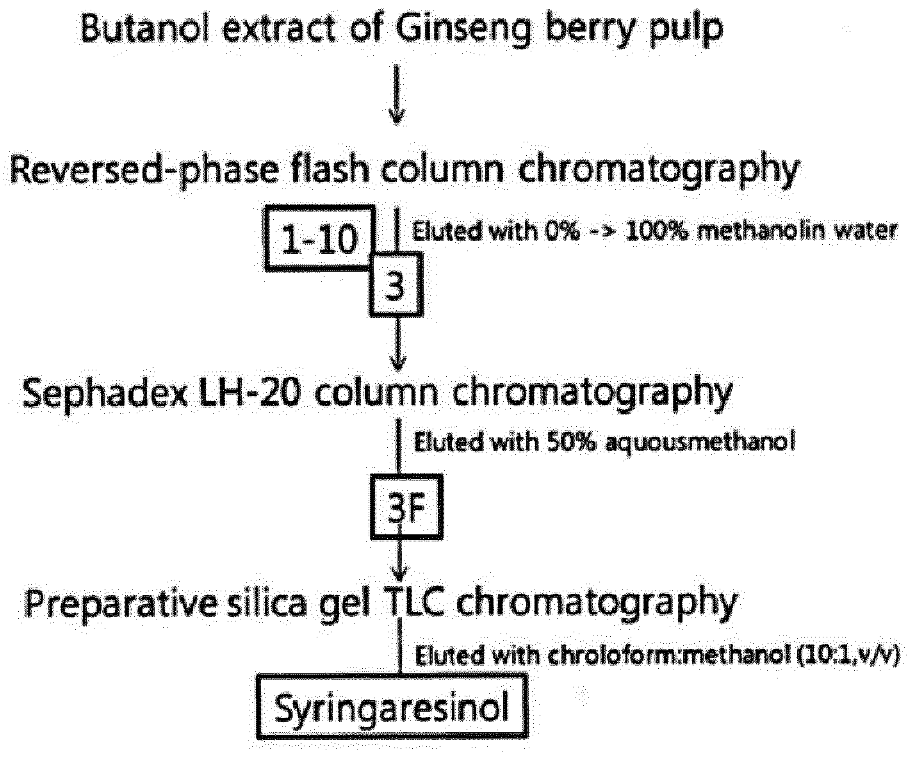
Butanol extract of Ginseng berry pulp
Reversed-phase flash column chromatography
1-10
3
Eluted with 0% -> 100% methanolin water
Sephadex LH-20 column chromatography
Eluted with 50% aquousmethanol
3F
Preparative silica gel TLC chromatography
Eluted with chroloform:methanol (10:1,v/v)
Syringaresinol

Fig.2

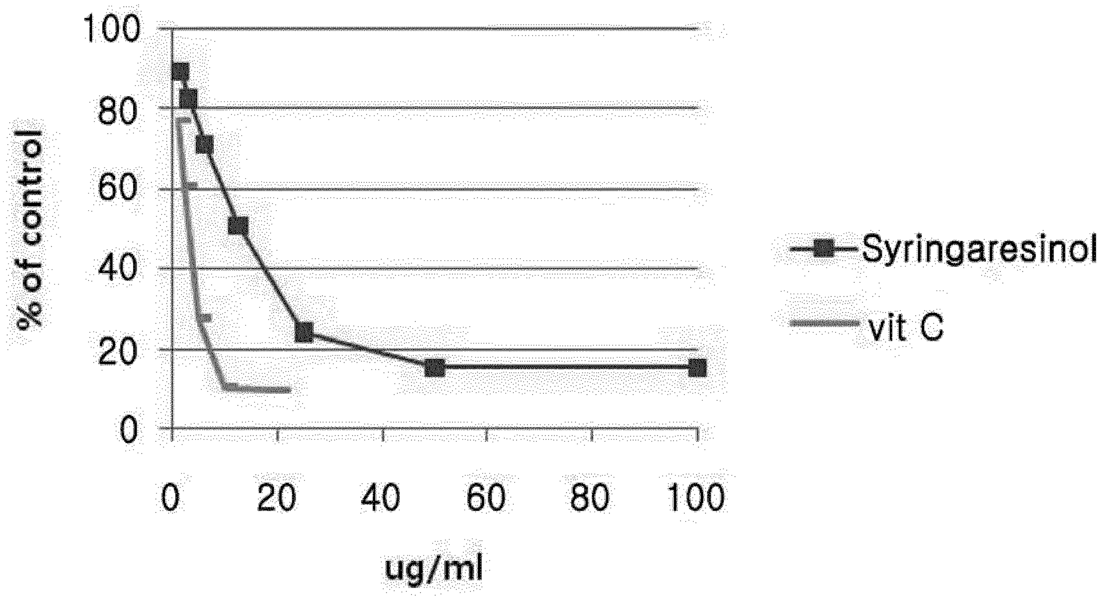
100
80
60
40
20
0
% of control
0
20
40
60
80
100
ug/ml
Syringaresinol
vit C

Fig.3

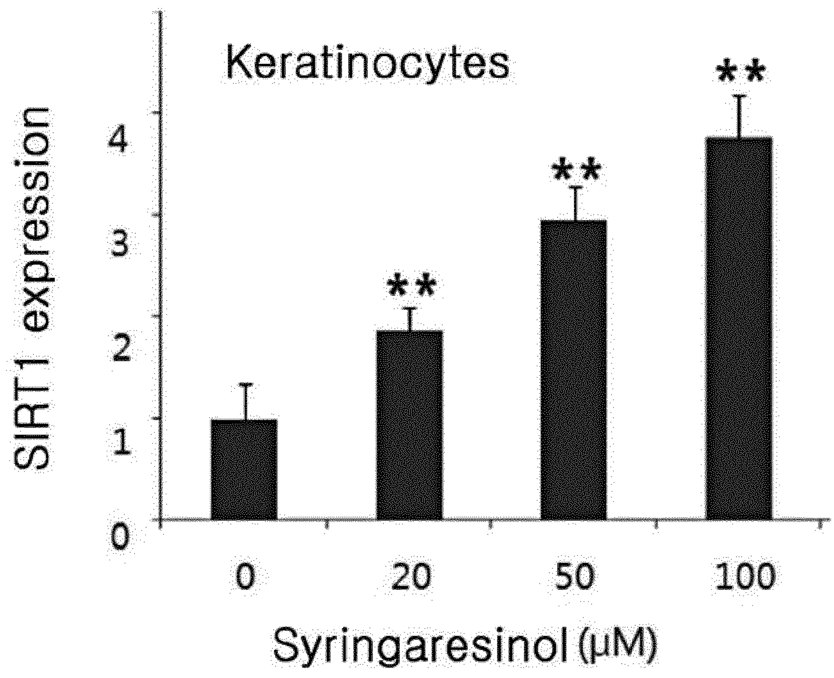
Keratinocytes
SIRT1 expression
0
1
2
3
4
**
**
**
0
20
50
100
Syringaresinol (μM)

Fig.4

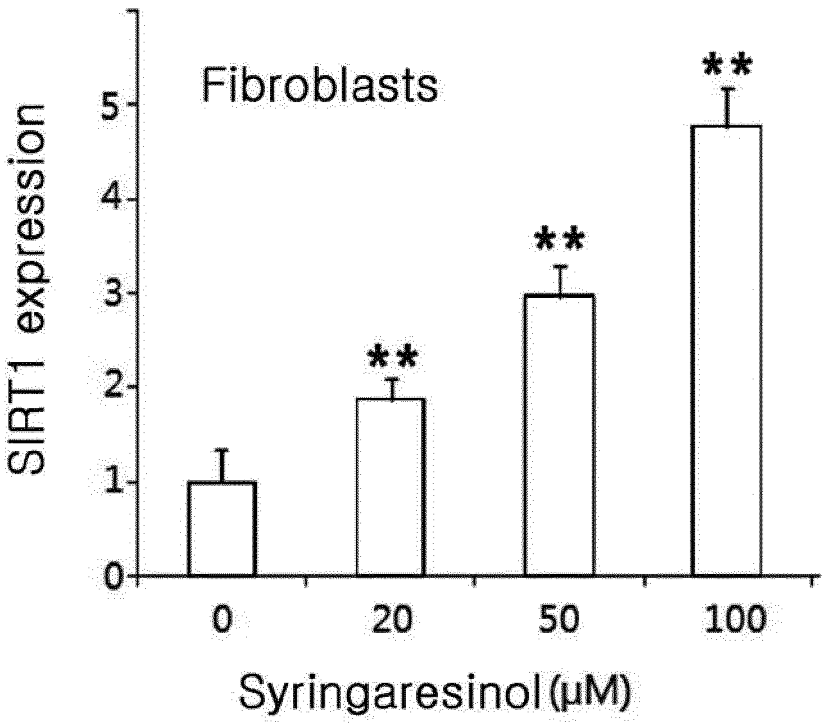
Fibroblasts
SIRT1 expression
0
1
2
3
4
5
**
**
**
0
20
50
100
Syringaresinol (μM)

Fig.5

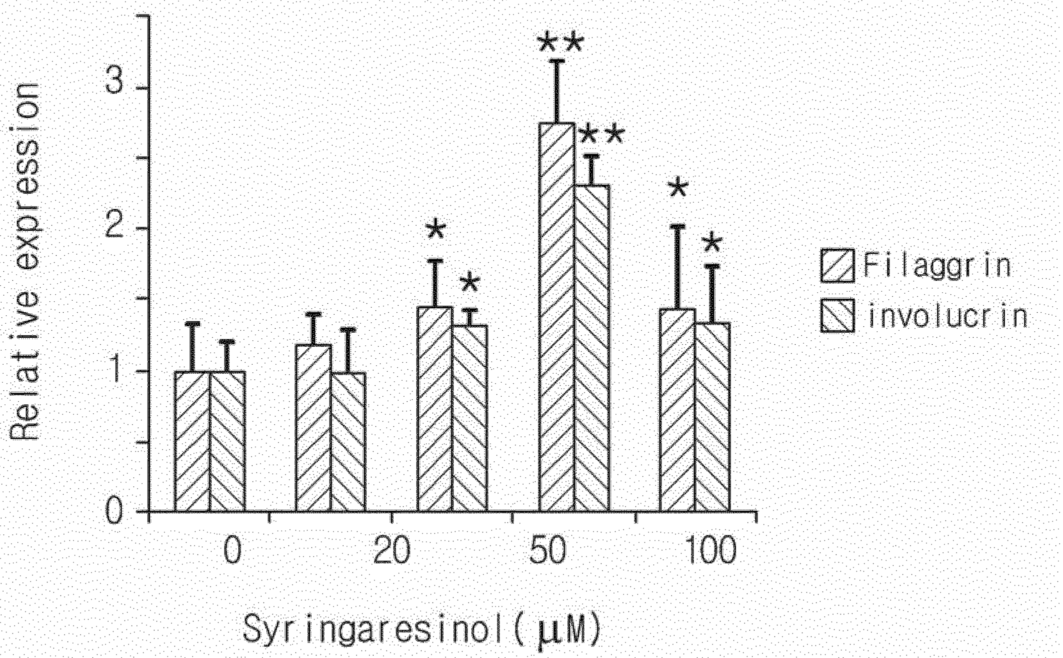
Relative expression
0
1
2
3
**
**
*
*
*
*
0
20
50
100
Syringaresinol(μM)
Filaggrin
involucrin

Fig.6

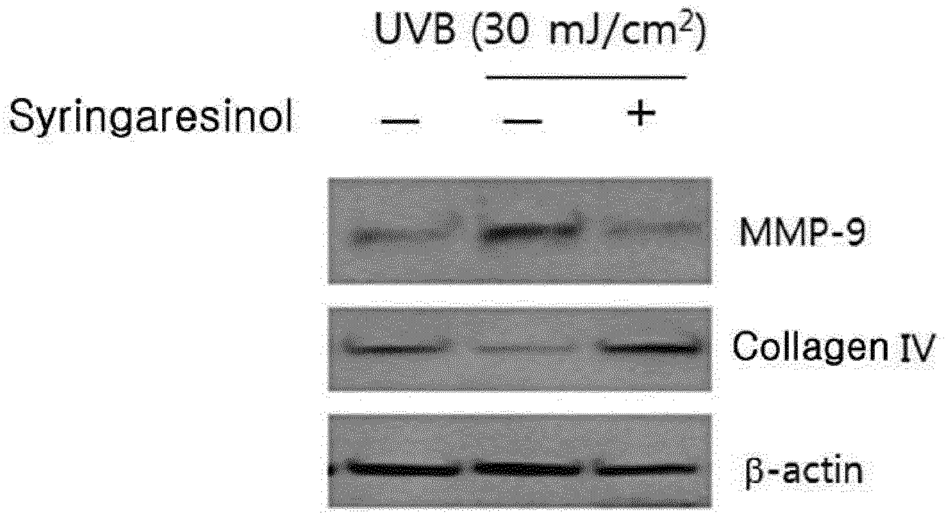

Fig.7

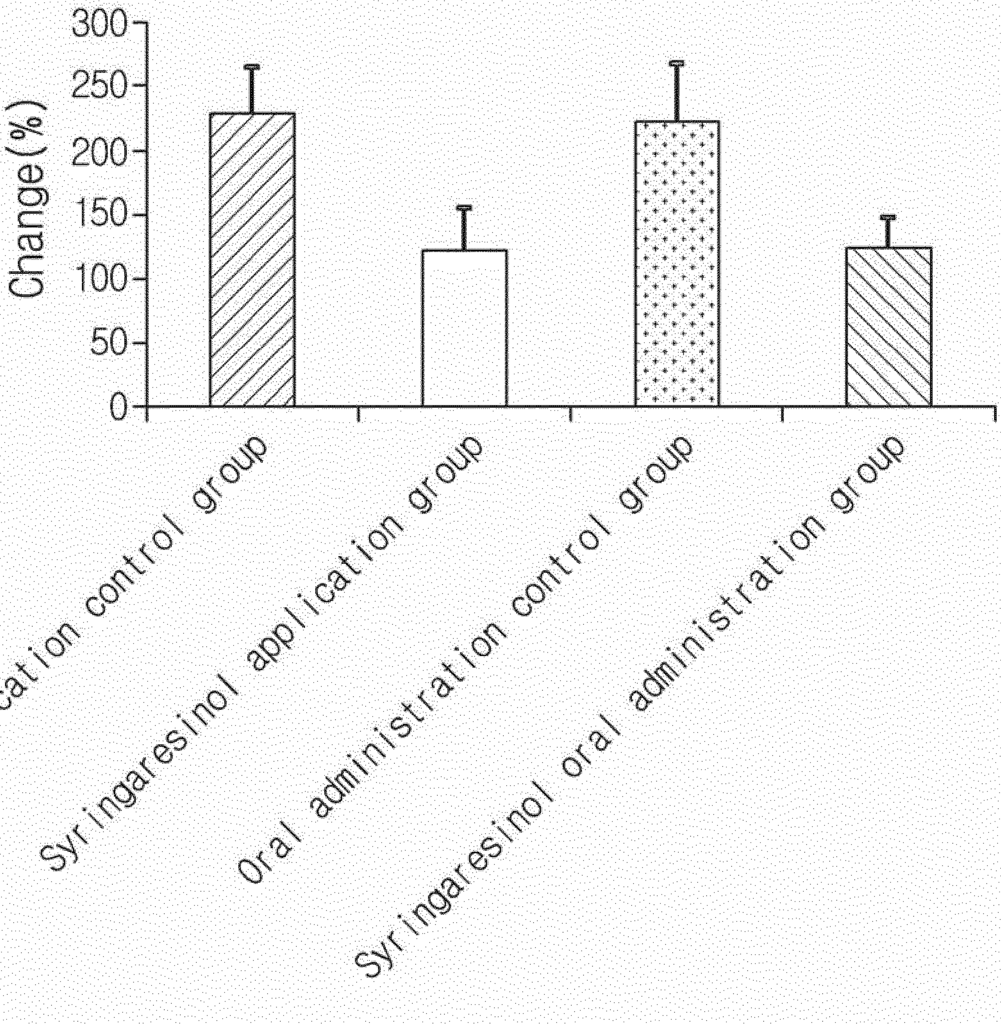
Change(%)
300
250
200
150
100
50
0
Application control group
Syringaresinol application group
Oral administration control group
Syringaresinol oral administration group

Fig.8

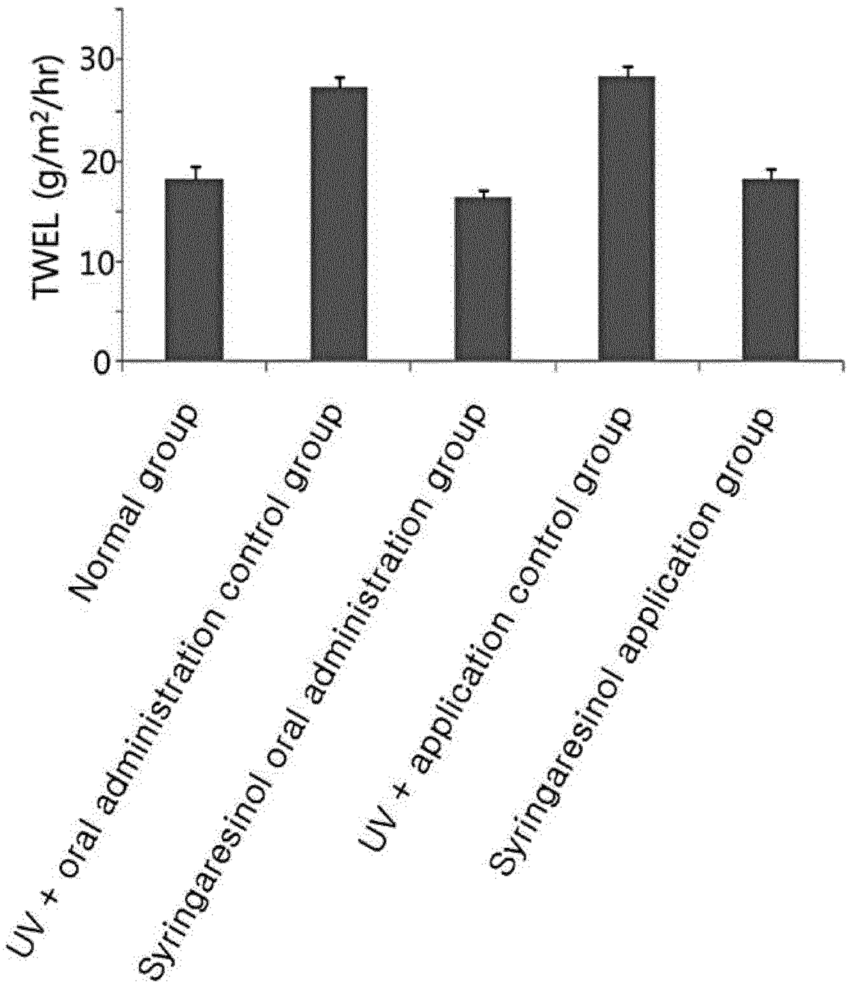
TWEL (g/m²/hr)
30
20
10
0
Normal group
UV + oral administration control group
Syringaresinol oral administration group
UV + application control group
Syringaresinol application group

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005048970 A
- JP 2011102272 A
- WO 2004000304 A1
- DE 10218476 A1
- US 2009130154 A1
- US 2007166255 A1
- JP 2009067718 A
- JP H0913031 B


### Non-patent literature cited in the description


- **KI HYUN KIM et al.** Lignans from the Tuber-barks of Colocasia antiquorum var. esculenta and their antimelanogenic activity. *Journal of agricultural and food chemistry,* 28 April 2010, vol. 58 (8), 4779-4785
- **WANG H.-M. et al.** Bioconstituents from stems of Synsepalum dulcificum Daniell (Sapotaceae) inhibit human melanoma proliferation, reduce mushroom tyroninase activity and have antioxidant properties. *Journal of the Taiwan institute of chemical engineers,* March 2011, vol. 42, 204-211
- **YAMAZAKI et al.** +)-Syringaresinol-di-O-beta-D-glucoside, a phenolic compound from Acanthopanax senticosus Harms, suppresses proinflammatory mediators in SW982 human synovial sarcoma cells by inhibiting activating protein-1 and/or nuclear factor-kB activities. *Toxicology in vitro,* 2007, vol. 21, 1530-1537